# EUROPEAN PATENT SPECIFICATION

(11) **EP 0 467 345 B1**
(45) Date of publication and mention of the grant of the patent: **21.05.1997**
(21) Application number: 91111972.5
(22) Date of filing: 17.07.1991
(51) Int. Cl.: C07C 2/26, C07C 2/74, C07C 9/22, C10M 127/02, C07C 2/20, C10M 107/10

(54) **Olefine oligomerization process and products and use of dimer products**
Olefinoligomerisierungsverfahren und Produkte und Anwendung von Dimerprodukten
Procédé d'oligomérisation d'oléfines et produits et utilisation des produits dimères

(30) Priority: 19.07.1990 US 554727; 12.04.1991 US 684291; 28.05.1991 US 705960
(43) Date of publication of application: 22.01.1992
(73) Proprietor: AMOCO CORPORATION, Chicago, IL 60601 (US)
(72) Inventor: Theriot, Kevin Jude, Baton Rouge, Louisiana 70808 (US); Shubkin, Ronald Lee, Baton Rouge, Louisiana 70817 (US)
(74) Representative: Schwabe - Sandmair - Marx

(56) References cited:
- EP-A- 0 352 723
- US-A- 2 806 072
- US-A- 4 172 855
- US-A- 4 434 309
- US-A- 4 490 571

## Description

This invention relates to α-olefin oligomers having utility as synthetic lubricants and functional fluids and more particularly to (1) the use of alcohol alkoxylates as promoters for BF₃ in the oligomerization of olefins to control the product distribution and provide higher percentages of lower oligomers, (2) α-olefin dimer products having improved low temperature properties which can be prepared by the oligomerization process, and (3) oil compositions containing the α-olefin dimer products.

As disclosed in U.S. 2,500,161 and 2,500,163, α-olefin oligomers and their use as synthetic lubricants, or "synlubes," are well known. The oligomers are usually hydrogenated to improve their stability, and the particular applications in which they are used depend upon their viscosities. The oligomer oils having viscosities of 2-10 mm²·s⁻¹ at 100°C, which are mixtures of different percentages of dimer, trimer, tetramer, pentamer, and higher oligomers, are preferred for general lubricating oil applications, while low viscosity (e.g., 1-3 mm²·s⁻¹ at 100°C) are especially useful in heat transfer, insulating, hydraulic, and low temperature lubricant applications.

It is known to use a BF₃-promoter catalyst system in the preparation of the olefin products. Promoters which have been taught to be useful include the water, carboxylic acid, alkyl halide, alcohol, and ether promoters of U.S. 2,766,312, the dialkylether of U.S. 2,806,072, the acid anhydrides, esters, ketones, and aldehydes of U.S. 3,382,291, and the ethylene glycol monomethyl ether, propylene glycol monoethyl ether, and diisobutyl ether of U.S. 4,172,855.

US-A-4 434 309 discloses a process which comprises contacting an α-olefin monomer containing 4 to 6 carbon atoms with the catalyst comprising boron trifluoride and an organic, protonic promoter. These promoters are disclosed to be alcohols and carboxylic acids. A specific example of such a promoter is diglycolamine (2-(2-amino-ethoxy)ethanol). At a temperature from 25 to 75°C under a pressure of 0 to 1000, a product which is predominant in dimer is obtained by use of primary alcohols and carboxylic acids as the promoter.

The various α-olefin oligomers are produced in different proportions in the oligomerization process. When increased viscosity is desired, processes are used which produce more of the higher oligomers, or some of the lower oligomers are removed from the product. Most low viscosity dimer and trimer products are obtained as by-products of the preparation of higher viscosity synthetic oils. Higher temperatures can be used to favor dimer production, but utilization of such higher temperatures can cause corrosion of production equipment. It has now been found that dimers can be produced as the primary oligomerization product at moderate temperatures by the process of this invention.

This invention provides:
(1) A process which comprises contacting an α-olefin monomer containing 6-20 carbons with a catalyst comprising boron trifluoride and an alcohol alkoxylate so as to form an oligomer product wherein the alcohol alkoxylate has the formula RO(CHR'-CHR''-(CHR''')ₘ-O)ₙH where m is 0, 1 or 2, R is hydrocarbyl containing 1-24 carbons, including mixtures thereof, R', R'', ad R''' are independently hydrogen, methyl, or ethyl and, when m is 2, each R'' can be different, and n averages 1-15.
(2) a 1-octene and/or 1-decene dimer and/or a 1-octene and 1-decene co-dimer oil composition comprising a mixture of isomers which in the case of the 1-octene dimer are C₁₆H₃₂ isomers containing less than about 2.5 weight % of 7-methylpentadecene isomers, in the case of the 1-decene dimer are C₂₀H₄₀ isomers containing less than about 2.5 weight % of 9-methylnonadecene isomers, and in the case of the co-dimer are C₁₈H₃₆ isomers containing less than about 2.5 weight % of the 7-methylheptadecene and/or 9-methylheptadecene isomers,
(3) a hydrogenated 1-octene and/or 1-decene dimer and/or 1-octene/1-decene co-dimer oil composition comprising a mixture of isomers which in the case of the 1-octene dimer are C₁₆H₃₄ isomers containing less than about 2.5 weight % of 7-methylpentadecane isomer, in the case of the 1-decene dimer are C₂₀H₄₂ isomers containing less than about 2.5 weight % of 9-methylnonadecane isomer, and in the case of the co-dimer are C₁₈H₃₆ isomers containing less than about 2.5 weight % of the 7-methylheptadecane and 9-methylheptadecane isomers,
(4) a heat transfer, lubricating, functional, and/or insulating fluid composition comprising (a) a dimer, co-dimer, hydrogenated dimer, or hydrogenated co-dimer of paragraph #2 or #3 above and (b) 0.1-25 weight % of the fluid composition of one or more oil additives, and
(5) Use of said fluid composition in heat transfer, lubricating, functional and/or insulating fluid applications.

The olefins used in making the oligomers in general are predominately (at least 50 mole %) C₆-C₂₀ straight-chain monoolefinically unsaturated hydrocarbons in which the olefinic unsaturation occurs at the 1- or α-position of the straight carbon chain. Examples of such olefins are 1-hexene, 1-heptene, 1-octene, 1-nonene, 1-decene, 1-dodecene, 1-hexadecene, 1-tetradecene, and mixtures thereof. The more preferred normal-α-olefin monomers are those containing 8-12 carbon atoms. The most preferred olefin monomers are 1-octene and 1-decene.

The olefin monomers can also contain minor amounts of up to about 50 and usually less than 25 mole percent of internal olefins and vinylidene olefins.

The olefin is contacted as known in the art with a catalytic amount of boron trifluoride which should be at least about 0.002 mole per mole of olefin. Preferably the reaction mixture is saturated with BF₃. To be effective, the boron trifluoride is used in combination with a promoter which is an alcohol alkoxylate. This promoter surprisingly favors the production of lower oligomers and particularly products containing predominantly dimer and trimer with a dimer to trimer ratio of greater than about 1. Under ordinary reaction conditions the dimer does not further react, and particularly does not dimerize, to any significant extent so that the reaction is easily controllable to produce a large proportion (at least about 40 and preferably 50 to 85 wt % or more dimer based on the total weight of oligomers in the product) of dimer. The dimer content asymptotically approaches a maximum rather than sharply peaking at a transient maximum, which is common in prior processes.

Alcohol alkoxylates useful in the invention are represented by the formula:

RO(CHR'-CHR''-(CHR''')ₘ-O)ₙH

where m is 0, 1 or 2, R is hydrocarbyl containing from 1 to 24 carbons, including mixtures thereof, R', R'' and R''' are independently hydrogen, methyl, or ethyl and when m is 2, each R''' can be different, and n averages 1 to 15.

Examples of such alcohol alkoxylates include glycol ethers such as ethylene glycol monomethyl ether (2-methoxyethanol) and propylene glycol monoethyl ether and ethoxylates derived from mixed C₂ to C₂₄, preferably C₂ to C₁₈ and most preferably C₆ to C₁₂ straight chain alcohols. Suitable ethoxylates where R' and R'' are hydrogen and n in the formula averages about 2 to 12, and preferably 3 to 6, are commercially available under the Ethonic® trademark.

The promoters are used in minor, effective amounts, for example, 0.001 to 0.040 mole per mole of α-olefin monomer (0.1 to 4.0 mole %). In general, the BF₃ is used in molar excess to the amount of promoter. This can be accomplished by using a closed reactor and a small BF₃ pressure over the reaction mixture. The promoter can be mixed with the olefin feed and the reaction can be carried out in a batch or continuous process at temperatures of 0° to 200°C and pressures ranging from atmospheric up to, e.g., 6.9 MPa. The reaction temperature will change the oligomer distribution with temperatures of about 50°C and above favoring the production of lower oligomers, namely dimer. Preferred reaction temperatures and pressures are 20° to 65°C and 5 to 100 psig (0.03-0.69 MPa).

The oligomer mixture from the reaction contains monomer which can be removed by distillation. The monomer has been found to contain mostly less reactive, isomerized material. However, this monomer can be recycled because it will react to form oligomers in the presence of fresh α-olefin monomer. For example, portions of up to about 25 wt % and preferably 5 to 15 wt % recycled monomer based on total monomer can be mixed with fresh monomer. The product mixture can be further separated by distillation to provide one or more product fractions having the desired viscosities for use in various lubricant applications such as drilling, hydraulic or metal working fluids, gear oils and crankcase lubricants.

The dimer products of the invention are a complex mixture of 25 or more isomers (a C₂₀H₄₂ paraffin can theoretically have 366,319 possible isomers). There is no absolute method for predicting the isomers most likely to be found in the hydrog-enated dimer products of 1-decene products. Accordingly, analysis of all of the isomers by gas chromatography is not practical, especially since peaks can be mixtures of closely related isomers (see Onopchenko, et al., "BF₃-Catalyzed Oligomerization of Alkenes: Structures, Mechanisms, and Properties", Ind. Eng. Chem. Prod. Res. Dev. 1983, 22, 182-191). However, it is possible to identify the more linear isomers such as those having no branches or a single branch with one carbon atom. The dimer products prepared using the alcohol alkoxylate promoters have improved low temperature properties because of their unique isomer content in that they contain less than about 2.5 weight percent and, especially, less than about 0.5 weight percent of relatively linear isomers.

The alcohol alkoxylates in the presence of BF₃, form stable complexes which separate from the product mixture on standing and can be readily recovered and reused. This avoids the BF₃ separation and recovery procedures necessary when using, for example, a BF₃-butanol complex. In fact, because the alcohol ethoxylates are surfactants, it is preferable to let the catalyst settle from the reaction mixture prior to quenching with base, and especially when using NaOH, in order to avoid the formation of an emulsion.

The oligomer product can be hydrogenated by conventional methods. Supported nickel catalysts are useful. For example, nickel on a kieselguhr support gives good results. Batch or continuous processes can be used. For example, the catalyst can be added to the liquid and stirred under hydrogen pressure or the liquid may be trickled through a fixed bed of the supported catalyst under hydrogen pressure. Hydrogen pressures of 100 to 1,000 psig (0.69-6.9 MPa) at temperatures of 150° to 300°C are especially useful.

The invention is further illustrated by the following examples in which the oligomerizations are performed in a three pint stirred reactor consisting of a glass reactor bowl, glass jacket, and a stainless steel top. The reactor is equipped with an air driven magnetic drive stirrer with a marine propeller, a heating/ cooling coil and circulating system, dip tube, gas inlet and outlet valves and a pressure relief valve.

### Examples 1-5

1-Decene (600.0 grams, 4.29 moles) and 1.0 mole % based on 1-decene of Ethonic 610-3, (which is a C₆ to C₁₀ mixed alcohol ethoxylate having an average of three (CH₂-CH₂O) groups), are charged into the reactor which is then assembled and purged with N₂ with gentle agitation for 30 minutes. During this time the reactor is brought up to the appropriate reaction temperature by the heating coil circulating system. The reactor is then pressurized (N₂) to 20 psig (0.14 MPa) to insure that no leaks exist. After the pressure is relieved the stirring rate is increased and BF₃ is introduced into the reactor via a sparge tube located below the surface of the liquid. After a brief (5-10 seconds) purge, the system is pressurized to 10 psig (0.07 MPa) with BF₃. The reaction is stopped after the chosen reaction time by venting the BF₃ through a 10 wt% NaOH scrubber and quenching with either 5% aqueous NaOH (Examples 2 and 3) or saturated Na₂SO₄ (Examples 1,4 and 5) (50-150 ml). The reactor is purged with dry N₂ until all of the BF₃ is removed. The polyalphaolefin (PAO) - unreacted decene mixture is washed several times with water, dried over anhydrous CaCl₂, and filtered. The product content is determined by gas chromatographic analysis. The reaction times, temperatures and product analysis are given in Table 1.

**TABLE 1**

| Example | Time (min) | Temperature (°C)[Max.] | GC Area %¹ Monomer | Dimer | Trimer | Tetramer |
|---|---|---|---|---|---|---|
| 1 | 120 | 20 [28] | 11 | 41 | 42 | 6 |
| 2 | 120 | 32 [39] | 15 | 48 | 32 | 4 |
| 3 | 120 | 45 [50] | 23 | 48 | 20 | 9 |
| 4 | 60 | 45 [52] | 29 | 50 | 19 | 3 |
| 5 | 120 | 80 [86] | 14 | 68 | 16 | 3 |

| | | | | | | |
|---|---|---|---|---|---|---|
| ¹Where area % ∼ weight % | | | | | | |

### Example 6

The process of Example 2 is repeated except at double the amount of alcohol ethoxylate (4 wt %/2 mole %) and quenching is with saturated Na₂SO₄. The product distribution in G.C. area % is 9% monomer, 48% dimer, 37% trimer and 6% tetramer.

### Example 7

The process of Example 3 is repeated except that quenching is with saturated Na₂SO₄ and 9.1 wt % of the decene monomer is recycled, considerably isomerized monomer from a previous reaction. The product distribution in G.C. area % is 20% monomer, 52% dimer, 24% trimer and 5% tetramer.

### Example 8

The process of Example 2 is repeated except that Ethonic 810-6 (2.8 wt %, 1.0 mole %) which is a C₈ to C₁₀ mixed alcohol ethoxylate having an average of six (CH₂-CH₂O) groups is used as the promoter and quenching is with saturated Na₂SO₄. The product distribution in G.C. area % is 24% monomer, 46% dimer, 26% trimer and 4% tetramer.

The dimer fractions from Examples 1, 2 and 3 are separated by distillation and hydrogenated. Their physical properties are reported in Table 2 where the composition is given in G.C. area %.

**TABLE 2**

| Example | 1 | 2 | 3 |
|---|---|---|---|
| Monomer | -- | 0.5 | 0.7 |
| Dimer | 98.9 | 96.7 | 97.9 |
| Trimer | 1.1 | 2.6 | 1.4 |
| Tetramer | -- | 0.2 | -- |
| V_{100°C}(cSt) | 1.71 | 1.66 | 1.63 |
| V_{40°C} (cSt) | 5.22 | 4.99 | -- |
| V_{-40°C} (cSt) | 266.0 | 251.0 | 257.0 |
| Pour Point (°C) | <-65 | <-65 | <-65 |
| Flash Point (°C) | 160.0 | 148.0 | 152.0 |

### Example 9 A-E

This example illustrates the recycle of the promoter/ BF₃ co-catalyst complex.

1-Decene (600.0 g, 4.29 mol) and Ethonic® 610-3 ethoxylate (11.79 g, 42.9 mmol) are charged into the reactor which is then assembled and purged with N₂ with gentle agitation for 30 minutes; during this time the vessel temperature is brought up to 45°C. The reactor is then pressurized (N₂) to 20 psig (0.14 MPa) to insure that no leaks exist. After the pressure is relieved, the stirring rate is increased and BF₃ is introduced into the reactor via a sparge tube located below the surface of the liquid. After a brief (5-10 seconds) purge, the system is pressurized to 10 psig (0.07 MPa) with BF₃. Periodic samples are collected and quenched with saturated aqueous Na₂SO₄, washed with water (twice), dried over anhydrous CaCl₂, filtered through syringe disk filters, and analyzed by gas chromatography.

After 60 minutes, the BF₃ is purged from the reactor with N₂ for about 30 minutes. The stirring is then stopped to allow the two existing phases to separate (∼20 minutes). The upper layer (product 9A) is then drained and washed with 5% aqueous NaOH followed by 2 water washes. The lower layer (co-catalyst) remains in the reactor.

At this point more 1-decene is added and a second reaction initiated by pressurizing the reactor with BF₃ (no additional Ethonic® 610-3 is added). After 60 minutes the mixture is again purged with N₂, allowed to settle (∼20 minutes), and the PAO drained (9B). This procedure is repeated once more to collect a third lot of PAO (9C).

After the third run, the co-catalyst layer is kept in the reactor under an atmosphere of BF₃/N₂. After 20 hours another run (120 minutes) is made to collect a fourth lot of PAO (9D). Again, after an additional 20 hours, a fifth run is made (9E). Results are tabulated in Table 3.

**TABLE 3**

| Reaction | Time (min) | GC Area% Monomer | Dimer | Trimer | Tetramer |
|---|---|---|---|---|---|
| 9A | 60 | 26 | 50 | 21 | 3 |
| 9B | 60 | 44 | 42 | 12 | 1 |
| 9C | 60 | 46 | 41 | 12 | 1 |
| 9D | 120 | 29 | 54 | 16 | 2 |
| 9E | 120 | 34 | 50 | 14 | 1 |

The results illustrate that the co-catalyst can be easily recycled and remains effective in providing high yields of dimer.

### Example 10

Example 3 is repeated using 2-methoxyethanol promoter at a concentration of 1 mole % based on monomer. After two hours the G.C. area % product distribution is 8% monomer, 77% dimer, 13% trimer and 2% tetramer or about 85% dimer based on total oligomer product with a conversion to oligomer of over 90%. Repeating the process at double the promoter concentration 2.0 mol % (1.0 wt %) gave about the same result in half the time (one hour instead of two). This example illustrates that an oligomer which is close to a 2 mm²·s⁻¹ (at 100°C) viscosity product can be produced by merely removing the monomer.

### Comparison

A product prepared from 1-decene monomer using a BF₃·n-butanol catalyst (1.3 mole % n-butanol on monomer) at a reaction temperature of 40°C and 20 psig (0.14 MPa) BF₃ pressure typically gives a G.C. area % product distribution of about 1% monomer, 2% dimer, 53% trimer, 28% tetramer, 11% pentamer, and 5% hexamer.

Samples of eicosane (linear C₂₀) and 9-methylnonadecane were obtained. The 9-methylnonadecane was synthesized by the dimerization of 1-decene using tri-n-octyl aluminum followed by hydrogenation. This procedure is known to give 9-methylnona-decane as the predominant C₂₀ product. A sample of 2-methoxy-ethanol generated hydrogenated decene dimer prepared according to Example 10 was spiked with eicosane and 9-methylnonadecane and analyzed by gas chromatography. No 9-methylnona-decane isomer was detected (less than 0.5 weight percent) in the unspiked sample. The retention time of the 9-methylnonadecane was the same as the relatively linear isomer in the 1-butanol/1-propanol generated material. Subsequently, GC/MS analysis confirmed that this isomer was 9-methylnona-decane. No eicosane was observed in either product.

Samples of the 2-methoxyethanol generated dimer were then spiked with varying amounts of 9-methylnonadecane and the KV_{-54°C} values in mm²·s⁻¹ determined with the results shown below:

**TABLE 4**

| Entry | % Isomer Added | KV_{-54°C} (20 min) | KV_{-54°C} Final | Comments |
|---|---|---|---|---|
| A | 0% | 1030 | 1030 | Clear, no vis. change |
| B | 1% | 1070 | 1060 | Sl. cloudy, 1.5 hour |
| C | 3% | 2110 | 1160 | Cloudy - 15 minutes |
| D | 5% | 1790 | 1200 | Cloudy - 10 minutes |
| Comparison | 0% | 1470 | 1220 | Cloudy |

| | | | | |
|---|---|---|---|---|
| ¹ - at 30 minutes | | | | |
| ² - at 1 hour | | | | |

Entries C and D behaved similarly to decene dimer PAO samples derived from 1-butanol/1-propanol co-catalyzed reactions (see "Comparison" in Table 4 which is a so-called "super low temperature" hydrogenated dimer derived from a BF₃/1-propanol catalyzed reaction), i.e., the KV_{-54°C} values were initially high and gradually decreased with time while the mixture became cloudy. The cloud point of a commercially available 2.0 mm²·s⁻¹ PAO fluid, which consisted of very pure decene dimer, was -57°C compared to a cloud point of <-70°C for a 2-methoxyethanol generated dimer of the invention. These results demonstrate that the dimer products of the invention have significantly improved physical properties caused by a difference in composition from prior 1-decene dimer compositions.

### Example 11

1-Octene (750.0 grams, 6.70 moles) and 2-methoxyethanol (5.09 grams, 67.0 moles) were charged into the reactor which was then assembled and purged with N₂ with gentle agitation for 30 minutes. During this time the vessel was heated to 45°C with the circulating system. The stirring rate was increased (rpm not measured) and BF₃ was introduced into the reactor via a sparge tube located below the surface of the liquid. After a brief (5-10 seconds) purge, the system was pressurized to 10 psig (0.07 MPa) with BF₃. An exotherm occurred which reached a maximum temperature of 64°C at ∼ 15 minutes.

The reaction was stopped by venting the BF₃ through a 10% NaOH scrubber and then quenching with 5% aqueous NaOH (100 mL). The reaction mixture was separated from the quenching medium, washed several times with water, allowed to settle, and filtered through filter paper.

The product contained 5% C₈, 64% C₁₆, 25% C₂₄, and 6% C₃₂ (normalized gas chromatography area percent).

The crude oligomerization product (1200 g) was hydrogenated in a 2-liter autoclave using a Ni/Kieselguhr catalyst (48 g) at 200°C and 500 psig (3.4 MPa) H₂ for 2 hours. After cooling, the product was filtered through filter-aid to give the crude hydrogenated product. The distillation was carried out at 1.5 mm Hg and the fraction boiling at 107-120°C was collected to give 535 g (1071 g feed) of pure hydrogenated octene dimer. The physical properties were as follows:

| | |
|---|---|
| KV_{100°C} | 1.09 mm²·s⁻¹ |
| KV_{40°C} | 2.69 mm²·s⁻¹ |
| KV_{-40°C} | 71.0 mm²·s⁻¹ |
| Pour point | <-65°C |
| Flash point | 122°C |

The target specifications for an IEC 296 - Class III and III_{A} transformer fluid are:

| | |
|---|---|
| KV_{40°C} | 3.5 mm²·s⁻¹ max |
| KV_{-40°C} | 150 mm²·s⁻¹ max |
| Pour point | -60°C max |
| Flash point | 95°C min |

In such use the fluid is contained in an electrical apparatus, i.e. a power transformer. The fluid surrounds an electrical component in the apparatus and acts as an electrical insulating and heat removal medium.

The physical properties of the octene dimer exceeded the transformer fluid specifications and demonstrated excellent low temperature performance.

The dimer oils of the invention can be used neat in various heat transfer, insulating and lubricant applications, and when used in insulating, functional fluid and/or lubricating oil applications (e.g., electro erosion, high speed aluminum cold rolling, transformer and switchgear oil, shock absorber, brake, hydraulic textile covering and spindle fluids, and drilling muds), their properties can be enhanced by the use of conventional oil additives in total amounts of up to about 25 weight percent and preferably 0.1 to 20 weight percent. Such additives include, for example, dispersants, antioxidants, anti-wear agents, anti-foam, corrosion inhibitors, detergents, and seal swell agents. These types of additives are well known in the art. Some examples of such additives are zinc dialkyldithiophosphates, calcium aryl sulfonates, overbased calcium aryl sulfonates, barium phenates, barium oxide neutralized reaction products of phosphorus pentasulfide and terpenes or high molecular weight olefins, hindered alkyl phenols, methylene-bis-dialkylphenols, dibutyl tin sulfide, dibutyl hydrogen phosphonate, tricresylphosphate, high molecular weight alkyl succinimides of ethylene-polyamines such as tetraethylenepolyamine, sulfur-bridged alkylphenols, sulfurized fatty acid esters and amides, silicones, and dialkylesters. Mixtures of the C₈ and C₁₀ dimer oils in any proportions can be used. The dimer compositions can contain minor amounts of higher oligomers (trimer, tetramer, and higher) but preferably contain at least 60 weight percent and more preferably at least 75 weight percent of dimers. The dimer oils can be used as additive oils or as base oils and in blends with mineral oils and with other synthetic oils such as, for example, synthetic esters.

## Claims

1. A process which comprises contacting an α-olefin monomer containing 6-20 carbons with a catalyst comprising boron trifluoride and an alcohol alkoxylate so as to form an oligomer product wherein the alcohol alkoxylate has the formula RO(CHR'-CHR''-(CHR''')ₘ-O)ₙH where m is 0, 1 or 2, R is hydrocarbyl containing 1-24 carbons, including mixtures thereof, R', R'', and R''' are independently hydrogen, methyl, or ethyl and, when m is 2, each R''' can be different, and n averages 1-15.

2. The process of claim 1 including hydrogenating the oligomer product.

3. The process of Claim 1 or 2 wherein one molar proportion of an α-olefin containing 8-12 carbons is contacted with a catalyst comprising at least 0.002 molar proportion of boron trifluoride and 0.001-0.040 molar proportion of the alcohol alkoxylate at a reaction temperature in the range of 0-200°C.

4. The process of any of the preceding claim wherein the alcohol alkoxylate is an ethoxylate of a mixture of straight chain alcohols containing 6-12 carbons and n averages 3-6.

5. The process of any of the preceding claims wherein the process is conducted at a reaction temperature and pressure of 20°-65°C and 0.03-0.69 MPa (5-100 psig).

6. The process of any of preceding claims which further comprises the step of separation of a product mixture by distillation.

7. The process according to claim 6 wherein separation by distillation is carried out to obtain a dimer product.

8. A 1-octene and/or 1-decene dimer and/or 1-octene and 1-decene co-dimer oil composition comprising a mixture of isomers which in the case of the 1-octene dimer are C₁₆H₃₂ isomers containing less than about 2.5 weight % of 7-methylpentadecene isomers, in the case of the 1-decene dimer are C₂₀H₄₀ isomers containing less than about 2.5 weight % of 9-methylnonadecene isomers, and in the case of the co-dimer are C₁₈H₃₆ isomers containing less than about 2.5 weight % of the 7-methylheptadecene and/or 9-methylheptadecene isomers.

9. A hydrogenated 1-octene and/or 1-decene dimer and/or 1-octene/1-decene co-dimer oil composition comprising a mixture of isomers which in the case of the 1-octene dimer are C₁₆H₃₄ isomers containing less than about 2.5 weight % of 7-methylpentadecane isomer, in the case of the 1-decene dimer are C₂₀H₄₂ isomers containing less than about 2.5 weight % of 9-methylnonadecane isomer, and in the case of the co-dimer are C₁₈H₃₈ isomers containing less than about 2.5 weight % of the 7-methylheptadecane and/or 9-methylnonadecane isomers.

10. A heat transfer, lubricating, functional, and/or insulating fluid composition which comprises (a) a mixture of 1-octene and/or 1-decene dimers and/or co-dimers of 1-octene and 1-decene which in the case of the 1-octene dimer are C₁₆H₃₂ isomers containing less than about 2.5 weight % of the 7-methylpentadecene isomers, in the case of the 1-decene dimer are C₂₀H₄₀ isomers containing less than about 2.5 weight % of the 9-methylheptadecene isomers, and in the case of the co-dimer are C₁₈H₃₆ isomers containing less than about 2.5 weight % of the 7-methylheptadecene and/or 9-methylheptadecene isomers, and (b) 0.1-25 weight % of fluid composition of one or more oil additives.

11. The fluid composition of claim 10 wherein the oil additives are selected from dispersants, antioxidants, anti-wear agents, anti-foam agents, corrosion inhibitors, detergents, and seal swell agents.

12. The fluid composition of claim 10 or 11 wherein the mixture of isomers is hydrogenated so as to provide C₁₆H₃₄ isomers containing less than about 2.5 weight % of 7-methylpentadecane and/or C₂₀H₄₂ isomers containing less than about 2.5 weight % of 9-methylnonadecane and/or C₁₈H₃₈ isomers containing less than about 2.5 weight % of 7-methylheptadecane and/or 9-methylheptadecane.

13. Use of the fluid composition according to any of claims 10 to 12 in heat transfer, lubricating, functional and/or insulating fluid applications.

## Patentansprüche

1. Verfahren, bei dem man ein α-Olefinmonomer mit 6 bis 20 Kohlenstoffatomen mit einem Bortrifluorid und ein Alkoholalkoxylat enthaltenden Katalysator in Kontakt bringt,um ein Oligomerprodukt herzustellen, in dem das Alkoholalkoxylat die Formel RO(CHR'-CHR''-(CHR''')ₘ-O)ₙH hat, in der m 0, 1 oder 2 ist, R Hydrocarbyl mit 1 bis 24 Kohlenstoffatomen einschließlich Mischungen davon bedeutet, R', R'' und R''' unabhängig voneinander Wasserstoff, Methyl oder Ethyl bedeuten, jedes R''' verschieden sein kann, wenn m 2 ist, und n im Durchschnitt 1 bis 15 beträgt.

2. Verfahren nach Anspruch 1, bei dem das Oligomerprodukt hydriert wird.

3. Verfahren nach Anspruch 1 oder 2, bei dem ein Molanteil eines α-Olefins mit 8 bis 12 Kohlenstoffatomen bei einer Reaktionstemperatur im Bereich von 0 bis 200°C mit einem Katalysator in Kontakt gebracht wird, der mindestens 0,002 Molanteile Bortrifluorid und 0,001 bis 0,040 Molanteile des Alkoholalkoxylats enthält.

4. Verfahren nach einem der vorstehenden Ansprüche, bei dem das Alkoholalkoxylat ein Ethoxylat oder eine Mischung geradkettiger Alkohole mit 6 bis 12 Kohlenstoffatomen ist und n im Durchschnitt 3 bis 6 beträgt.

5. Verfahren nach einem der vorstehenden Ansprüche, das bei einer Reaktionstemperatur von 20 bis 65°C und einem Druck von, 0,03 bis 0,69 MPa (5 bis 100 psig) durchgeführt wird.

6. Verfahren nach einem der vorstehenden Ansprüche, das zusätzlich den Schritt der Trennung einer Produktmischung durch Destillation umfaßt.

7. Verfahren nach Anspruch 6, in dem die Trennung durch Destillation durchgeführt wird, um ein Dimerprodukt zu erhalten.

8. Ölzusammensetzung aus einem Oct-1-en- und/oder Dec-1-endimer und/oder Oct-1-en- und Dec-1-encodimer, die eine Mischung von Isomeren enthält, bei denen es sich im Fall des Oct-1-endimeren um C₁₆H₃₂-Isomere mit weniger als etwa 2,5 gew.-% 7-Methylpentadecenisomeren, im Fall des Dec-1-endimeren um C₂₀H₄₀-Isomere mit weniger als etwa 2,5 Gew.-% 9-Methylnonadecenisomeren und im Fall des Codimeren um C₁₈H₃₆-Isomere mit weniger als etwa 2,5 Gew.-% der 7-Methylheptadecen- und/oder 9-Methylheptadecenisomeren handelt.

9. Hydrierte Ölzusammensetzung aus einem Oct-1-en- und/oder Dec-1-endimer und/oder Oct-1-en- und Dec-1-encodimer, die eine Mischung von Isomeren enthält,bei denen es sich im Fall des Oct-1-endimeren um C₁₆H₃₄-Isomere mit weniger als etwa 2,5 Gew.-% 7-Methylpentadecanisomer, im Fall des Dec-1-endimeren um C₂₀H₄₂-Isomere mit weniger als etwa 2,5 Gew.-% 9-Methylnonadecanisomer und im Fall des Codimeren um C₁₈H₃₈-Isomere mit weniger als etwa 2,5 Gew.-% der 7-Methylheptadecan- und/oder 9-Methylnonadecanisomeren handelt.

10. Wärmeübertragende, schmierende, funktionelle und/oder isolierende Fluidzusammensetzung, enthaltend (a) eine Mischung aus Oct-1-en- und/oder Dec-1-endimeren und/oder Oct-1-en- und Dec-1-encodimeren, bei denen es sich im Fall des Oct-1-endimeren um C₁₆H₃₂-Isomere mit weniger als etwa 2,5 Gew.-% 7-Methylpentadecenisomeren, im Fall des Dec-1-endimeren um C₂₀H₄₀-Isomere mit weniger als etwa 2,5 Gew.-% 9-Methylheptadecenisomeren und im Fall des Codimeren um C₁₈H₃₆-Isomere mit weniger als etwa 2,5 Gew.-% der 7-Methylheptadecen- und/oder 9-Methylheptadecenisomeren handelt, und (b) 0,1 bis 25 Gew.-% einer Fluidzusammensetzung aus einem oder mehreren Öladditiven.

11. Fluidzusammensetzung nach Anspruch 10, in der die Öladditive aus Dispergiermitteln, Antioxidantien, verschleißhemmenden Mitteln, schaumbremsenden Mitteln, Korrosionshemmern, Detergenzien und Dichtungsquellmitteln ausgewählt sind.

12. Fluidzusammensetzung nach Anspruch 10 oder 11, in der die Isomermischung hydriert wird, um C₁₆H₃₄-Isomere mit weniger als etwa 2,5 Gew.-% 7-Methylpentadecan, C₂₀H₄₂-Isomere mit weniger als etwa 2,5 Gew.-% 9-Methylnonadecan und/oder C₁₈H₃₈-Isomere mit weniger als etwa 2,5 Gew.-% 7-Methylheptadecan- und/oder 9-Methylheptadecan zur Verfügung zu stellen.

13. Verwendung der Fluidzusammensetzung nach einem der Ansprüche 10 bis 12 für Wärmeübertragungs- und Schmieranwendungen und/oder für Anwendungen als funktionelles und/oder isolierendes Fluid.

## Revendications

1. Procédé qui comprend la mise en contact d'un monomère d'α-oléfine contenant de 6 à 20 atomes de carbone avec un catalyseur comprenant du trifluorure de bore et un alkoxylate d'alcool, de manière à former un produit oligomère dans lequel l'alcoxylate d'alcool présente la formule RO(CHR'-CHR''-(CHR''')ₘ-O)ₙH, dans laquelle m représente 0, 1 ou 2, R représente un hydrocarbyle contenant de 1 à 24 atomes de carbone, y compris des mélanges de ceux-ci, R', R'' et R''' représentent indépendamment l'hydrogène, le méthyle ou l'éthyle et, lorsque m vaut 2, chaque R''' peut être différent, et n vaut en moyenne 1 à 15.

2. Procédé selon la revendication 1, comprenant l'hydrogénation du produit oligomère.

3. Procédé selon la revendication 1 ou 2, dans lequel une proportion molaire d une α-oléfine contenant de 8 à 12 atomes de carbone est mise en contact avec un catalyseur comprenant au moins une proportion 0,002 molaire de trifluorure de bore et une proportion 0,001 à 0,040 molaire de l'alcoxylate d'alcool, à une température de réaction située dans la plage de 0 à 200°C.

4. Procédé selon l'une quelconque des revendications précédentes, dans lequel l'alcoxylate d'alcool est un éthoxylate d'un mélange d'alcools à chaîne linéaire contenant de 6 à 12 atomes de carbone, et n vaut en moyenne 3 à 6.

5. Procédé selon l'une quelconque des revendications précédentes, dans lequel le procédé est conduit à une température de 20° à 65°C, et à une pression de 0,03 à 0,69 MPa (5 à 100 psig).

6. Procédé selon l'une quelconque des revendications précédentes, qui comprend en outre l'étape de séparation d'un mélange de produit par distillation.

7. Procédé selon la revendication 6, dans lequel la séparation par distillation est conduite pour obtenir un produit dimère.

8. Composition d'huile de dimère de 1-octène et/ou de 1-décène, et/ou de co-dimère de 1-octène et de 1-décène, comprenant un mélange d'isomères qui, dans le cas du dimère de 1-octène sont des isomères C₁₆H₃₂ contenant moins d'environ 2,5% en poids d'isomères de 7-méthylpentadécène, dans le cas du dimère de 1-décene, sont des isomères C₂₀H₄₀ contenant moins d'environ 2,5% en poids d'isomères de 9-méthylnonadécène et, dans le cas du co-dimère, sont des isomères C₁₈H₃₆ contenant moins d'environ 2,5% en poids des isomères de 7-méthylheptadécène et/ou de 9-méthylheptadécène.

9. Composition d'huile de dimère de 1-octène et/ou de 1-décène, et/ou de co-dimère de 1-octène et de 1-décène, comprenant un mélange d'isomères qui, dans le cas du dimère de 1-octène, sont des isomères C₁₆H₃₄ contenant moins d'environ 2,5% en poids d'isomère de 7-méthylpentadécane, dans le cas du dimère de 1-décène, sont des isomères C₂₀H₄₂ contenant moins d'environ 2,5% en poids d'isomères de 9-méthylnonadécane et, dans le cas du co-dimère, sont des isomères C₁₈H₃₈ contenant moins d'environ 2,5% en poids des isomères de 7-méthylheptadécane et/ou de 9-méthylheptadécane.

10. Composition de fluide de transfert thermique, de lubrification, fonctionnel et/ou isolant, qui comprend (a) un mélange de dimères de 1-octène et/ou de 1-décène et/ou de co-dimère de 1-octène et de 1-décène, qui, dans le cas du dimère de 1-octène, sont des isomeres C₁₆H₃₂ contenant moins d'environ 2,5% en poids des isomères de 7-méthylpentadécène, dans le cas du dimère de 1-décène, sont des isomères C₂₀H₄₀ contenant moins d'environ 2,5% en poids des isomères de 9-méthylheptadécène, et dans le cas du codimère sont des isomères C₁₈H₃₆ contenant moins d'environ 2,5% en poids des isomères de 7-méthylheptadécène et/ou de 9-méthylheptadécène, et (b) de 0,1 à 25% en poids de la composition de fluide de un ou plusieurs additifs pour huile.

11. Composition de fluide selon la revendication 10, dans laquelle les additifs pour huile sont choisis parmi des agents dispersants, des agents antioxydants, des agents antiusures, des agents antimousse, des inhibiteurs de corrosion, des détergents et des agents de gonflement des joints.

12. Composition de fluide selon les revendications 10 ou 11, dans laquelle le mélange d'isomères est hydrogéné de manière à fournir des isomères C₁₆H₃₄ contenant moins d'environ 2,5% en poids d'isomères de 7-méthylpentadécane et/ou de C₂₀H₄₂ contenant moins d'environ 2,5% en poids d'isomères de 9-méthylnonadécane et/ou de C₁₈H₃₈ contenant moins d'environ 2,5% en poids de 7-méthylheptadécane et/ou de 9-méthylheptadécane.

13. Utilisation de la composition fluide selon l'une quelconque des revendications 10 à 12 dans des applications de fluide de transfert thermique, de lubrification, fonctionnel et/ou isolant.
